# EUROPEAN PATENT APPLICATION

(11) **EP 3 778 888 A1**
(43) Date of publication of application: **17.02.2021**
(21) Application number: 19774261.2
(22) Date of filing: 28.03.2019
(51) Int. Cl.: C12N 15/09, A61K 31/7088, A61K 31/713, A61P 37/04, C12N 5/074, C12N 5/10

(54) **GENOME EDITING METHOD, COMPOSITION, CELL, CELL PREPARATION, AND METHOD FOR PRODUCING CELL PREPARATION**

(30) Priority: 29.03.2018 JP 2018066174
(71) Applicant: Jichi Medical University, Tokyo 102-0093 (JP)
(72) Inventor: HANAZONO, Yutaka, Tochigi 329-0498 (JP); HARA, Hiromasa, Tochigi 329-0498 (JP); UOSAKI, Hideki, Tochigi 329-0498 (JP)
(74) Representative: HGF
(86) International application number: PCT/JP2019/013602
(87) International publication number: WO 2019/189573

(57) **Abstract**

A genome editing method for an isolated cell, the method comprising introducing foreign DNA into a targeted genome with homologous recombination of at least one of a 5'-end or a 3'-end of the foreign DNA, where the foreign DNA has homology arms, each with a length of less than 500 bp.

SEQUENCE LISTING

<110>  Jichi Medical University

<120>  Method for gene editing, composition, cell, cell preparation and
       method for manufacturing a cell preparation

<130>  PC27495

<150>  JP2018-66174
<151>  2018-03-29

<160>  36

<170>  PatentIn version 3.5

<210>  1
<211>  10
<212>  DNA
<213>  Sus scrofa

<400>  1
ggcccaggtt                                                              10


<210>  2
<211>  50
<212>  DNA
<213>  Sus scrofa

<400>  2
caaaaggaaa tgtgtgggtg gggaggggta gtgggtaagg ggcccaggtt                  50


<210>  3
<211>  155
<212>  DNA
<213>  Artificial Sequence

<220>
<223>  Exon1 of IL2RG

<400>  3
cctgacacag tctacaccca ggaaacaagg agtaagcgcc atgctcaaac cccccctccc       60

cgtcaagtct ctcctcttcc tccagctccc tctgctcggc gtcggcctca atcctaaggt      120

cctcacccac agcggcaacg aggacatcac cgctg                                 155


<210>  4
<211>  50
<212>  DNA
<213>  Sus scrofa

<400>  4
gtgggaaact gggacgttgg gggtagggtt ggtgagccgg gggaggctgg                  50


<210>  5
<211>  10
<212>  DNA
<213>  Sus scrofa

<400>  5
gtgggaaact                                                              10


<210>  6
<211>  23
<212>  DNA
<213>  Artificial Sequence

<220>
<223>  IL2RG HITI

<400>  6
ccttcgggtt cagtcccacc cca                                                23


<210>  7
<211>  36
<212>  RNA
<213>  Artificial Sequence

<220>
<223>  IL2RG crRNA

<400>  7
ugggguggga cugaacccga guuuuagagc uaugcu                                  36


<210>  8
<211>  10
<212>  DNA
<213>  Mus musculus

<400>  8
tgcaactcca                                                              10


<210>  9
<211>  50
<212>  DNA
<213>  Mus musculus

<400>  9
tgggcctggg agaatccctt ccccctcttc cctcgtgatc tgcaactcca                  50


<210>  10
<211>  100
<212>  DNA
<213>  Mus musculus

<400>  10
aatacctttc tgggagttct ctgctgcctc ctggcttctg aggaccgccc tgggcctggg       60

agaatccctt ccccctcttc cctcgtgatc tgcaactcca                            100


<210>  11
<211>  10
<212>  DNA
<213>  Mus musculus

<400>  11
acaggtgtaa                                                              10


<210>  12
<211>  50
<212>  DNA
<213>  Mus musculus

<400>  12
acaggtgtaa aattggaggg acaagacttc ccacagattt tcggttttgt                  50


<210>  13
<211>  100
<212>  DNA
<213>  Mus musculus

<400>  13
acaggtgtaa aattggaggg acaagacttc ccacagattt tcggttttgt cgggaagttt      60

tttaataggg gcaaataagg aaaatgggag gataggtagt                            100


<210>  14
<211>  853
<212>  DNA
<213>  Artificial Sequence

<220>
<223>  GFP

<400>  14
actagttcta gcatctgtag ggcgcagtag tccagggttt ccttgatgat gtcatactta      60

tcctgtccct tttttttcca cagctcgcgg ttgaggacaa actcttcgcg catgcggatc     120

cggtaccatg gtgagcaagg gcgaggagct gttcaccggg gtggtgccca tcctggtcga     180

gctggacggc gacgtaaacg gccacaagtt cagcgtgtcc ggcgagggcg agggcgatgc     240

cacctacggc aagctgaccc tgaagttcat ctgcaccacc ggcaagctgc ccgtgccctg     300

gcccaccctc gtgaccaccc tgacctacgg cgtgcagtgc ttcagccgct accccgacca     360

catgaagcag cacgacttct tcaagtccgc catgcccgaa ggctacgtcc aggagcgcac     420

catcttcttc aaggacgacg gcaactacaa gacccgcgcc gaggtgaagt tcgagggcga     480

caccctggtg aaccgcatcg agctgaaggg catcgacttc aaggaggacg gcaacatcct     540

ggggcacaag ctggagtaca actacaacag ccacaacgtc tatatcatgg ccgacaagca     600

gaagaacggc atcaaggtga acttcaagat ccgccacaac atcgaggacg gcagcgtgca     660

gctcgccgac cactaccagc agaacacccc catcggcgac ggccccgtgc tgctgcccga     720

caaccactac ctgagcaccc agtccgccct gagcaaagac cccaacgaga agcgcgatca     780

catggtcctg ctggagttcg tgaccgccgc cgggatcact ctcggcatgg acgagctgta     840

caagtaagaa ttc                                                         853

<210>  15
<211>  23
<212>  DNA
<213>  Artificial Sequence

<220>
<223>  Rosa26 HITI

<400>  15
ccatcttcta gaaagactgg agt                                              23


<210>  16
<211>  36
<212>  RNA
<213>  Artificial Sequence

<220>
<223>  Rosa26 crRNA

<400>  16
acuccagucu uucuagaaga guuuuagagc uaugcu                                36


<210>  17
<211>  100
<212>  DNA
<213>  Mus musculus

<400>  17
ggatcggtgg ctccatcctg gcctcactgt ccaccttcca gcagatgtgg atcagcaagc      60

aggagtacga tgagtccggc ccctccatcg tgcaccgcaa                           100


<210>  18
<211>  100
<212>  DNA
<213>  Mus musculus

<400>  18
ggactgttac tgagctgcgt tttacaccct ttctttgaca aaacctaact tgcgcagaaa      60

aaaaaaaaat aagagacaac attggcatgg ctttgttttt                           100


<210>  19
<211>  23
<212>  DNA
<213>  Artificial Sequence

<220>
<223>  Actb HITI

<400>  19
agtccgccta gaagcacttg cgg                                              23


<210>  20
<211>  36
<212>  RNA
<213>  Artificial Sequence

<220>
<223>  Actb crRNA

<400>  20
aguccgccua gaagcacuug guuuuagagc uaugcu                                36


<210>  21
<211>  60
<212>  DNA
<213>  Homo sapiens

<400>  21
gatgaaccag gttatgacct tgatttattt tgcataccta atcattatgc tgaggatttg      60


<210>  22
<211>  244
<212>  DNA
<213>  Homo sapiens

<400>  22
ccggcctgtt gttttcttac ataattcatt atcataccta caaagttaac agttactaat      60

atcatcttac acctaaattt ctctgataga ctaaggttat tttttaacat cttaatccaa     120

tcaaatgttt gtatcctgta atgctctcat tgaaacagct atatttcttt ttcagattag     180

tgatgatgaa ccaggttatg accttgattt attttgcata cctaatcatt atgctgagga     240

tttg                                                                  244


<210>  23
<211>  61
<212>  DNA
<213>  Homo sapiens

<400>  23
gaaagggtgt ttattcctca tggactaatt atggacaggt aagtaagatc ttaaaatgag      60

g                                                                      61


<210>  24
<211>  239
<212>  DNA
<213>  Homo sapiens

<400>  24
gaaagggtgt ttattcctca tggactaatt atggacaggt aagtaagatc ttaaaatgag      60

gttttttact ttttcttgtg ttaatttcaa acatcagcag ctgttctgag tacttgctat     120

ttgaacataa actaggccaa cttattaaat aactgatgct ttctaaaatc ttctttatta     180

aaaataaaag aggagggcct tactaattac ttagtatcag ttgtggtata gtgggactc      239


<210>  25
<211>  732
<212>  DNA

<213>  Artificial Sequence

<220>
<223>  GFP

<400>  25
gaattcatgg tgagcaaggg cgaggagctg ttcaccgggg tggtgcccat cctggtcgag      60

ctggacggcg acgtaaacgg ccacaagttc agcgtgtccg gcgagggcga gggcgatgcc     120

acctacggca agctgaccct gaagttcatc tgcaccaccg gcaagctgcc cgtgccctgg     180

cccaccctcg tgaccaccct gacctacggc gtgcagtgct tcagccgcta ccccgaccac     240

atgaagcagc acgacttctt caagtccgcc atgcccgaag gctacgtcca ggagcgcacc     300

atcttcttca aggacgacgg caactacaag acccgcgccg aggtgaagtt cgagggcgac     360

accctggtga accgcatcga gctgaagggc atcgacttca aggaggacgg caacatcctg     420

gggcacaagc tggagtacaa cttcaacagc cacaacgtct atatcatggc cgacaagcag     480

aagaacggca tcaaggcgaa cttcaagatc cgccacaaca tcgaggacgg cagcgtgcag     540

ctcgccgacc actaccagca gaacaccccc atcggcgacg gccccgtgct gctgcccgac     600

aaccactacc tgaccaccca gtccgccctg agcaaagacc ccaacgagaa gcgcgatcac     660

atggtcctgg tggagttcgt gaccgccgcc gggatcactc tcggcatgga cgagctgtac     720

aagtaaggat cc                                                         732


<210>  26
<211>  26
<212>  DNA
<213>  Artificial Sequence

<220>
<223>  HPRT HITI

<400>  26
accctttcca aatcctcagc ataatg                                           26


<210>  27
<211>  20
<212>  RNA
<213>  Artificial Sequence

<220>
<223>  HPRT gRNA

<400>  27
uuaugcugag gauuuggaaa                                                   20


<210>  28
<211>  101
<212>  DNA
<213>  Homo sapiens

<400>  28

cgccggtttg tgccttcggt ccccgcttcg ccccctgccg tcccctcctt atcacggtcc 60

cgctcgcggc ctcgccgccc cgctgtctcc gccgcccgcc a 101

<210> 29
<211> 101
<212> DNA
<213> Homo sapiens

<400> 29
acccccgtgc cgccgcggat gggcagccgc gctggcggcc ccaccacgcc gctgagcccc 60

acgcgcctgt cgcggctcca ggagaaggag gagctgcgcg a 101

<210> 30
<211> 740
<212> DNA
<213> Artificial Sequence

<220>
<223> GFP

<400> 30
gatctgacaa tggtgagcaa gggcgaggag ctgttcaccg gggtggtgcc catcctggtc 60

gagctggacg gcgacgtaaa cggccacaag ttcagcgtgt ccggcgaggg cgagggcgat 120

gccacctacg gcaagctgac cctgaagttc atctgcacca ccggcaagct gcccgtgccc 180

tggcccaccc tcgtgaccac cctgacctac ggcgtgcagt gcttcagccg ctaccccgac 240

cacatgaagc agcacgactt cttcaagtcc gccatgcccg aaggctacgt ccaggagcgc 300

accatcttct tcaaggacga cggcaactac aagacccgcg ccgaggtgaa gttcgagggc 360

gacaccctgg tgaaccgcat cgagctgaag ggcatcgact tcaaggagga cggcaacatc 420

ctggggcaca agctggagta caacttcaac agccacaacg tctatatcat ggccgacaag 480

cagaagaacg gcatcaaggc gaacttcaag atccgccaca acatcgagga cggcagcgtg 540

cagctcgccg accactacca gcagaacacc cccatcggcg acggccccgt gctgctgccc 600

gacaaccact acctgaccac ccagtccgcc ctgagcaaag accccaacga gaagcgcgat 660

cacatggtcc tggtggagtt cgtgaccgcc gccgggatca ctctcggcat ggacgagctg 720

tacaaggccg gctccggtac 740

<210> 31
<211> 23
<212> DNA
<213> Artificial Sequence

<220>
<223> LMNB1 HITI

<400> 31
ggggtcgcag tcgccatggc ggg 23

<210>  32
<211>  23
<212>  DNA
<213>  Artificial Sequence

<220>
<223>  rcHITI

<400>  32
cccgccatgg cgactgcgac ccc                                              23


<210>  33
<211>  20
<212>  RNA
<213>  Artificial Sequence

<220>
<223>  LMNB1 gRNA

<400>  33
ggggucgcag ucgccauggc                                                  20


<210>  34
<211>  10
<212>  DNA
<213>  Homo sapiens

<400>  34
tgaggatttg                                                             10


<210>  35
<211>  10
<212>  DNA
<213>  Homo sapiens

<400>  35
gaaagggtgt                                                             10


<210>  36
<211>  36
<212>  RNA
<213>  Artificial Sequence

<220>
<223>  HPRT crRNA

<400>  36
uuaugcugag gauuuggaaa guuuuagagc uaugcu                                36

## Description

### [Technical Field]

The present invention relates to a genome editing method, a composition, a cell, a cell preparation, and a method for producing a cell preparation.

Priority is claimed on Japanese Patent Application No. 2018-66174, filed March 29, 2018, the content of which is incorporated herein by reference.

### [Background Art]

Homologous recombination is generally utilized when attempting to insert a specific gene into a specific site in a genome of an eukaryotic cell so that targeted genomic DNA is completely substituted by a desired base sequence. Specifically, a vector for gene transfer (hereinafter referred to as a targeting vector), which includes DNA (hereinafter referred to as a homology arm) having a sequence homologous to a site of a genome on which insertion is performed, has been used at both ends (5'-end and 3'-end) of foreign DNA to be inserted. When such a vector is introduced into a target cell, homologous recombination occurs between genomic DNA and the vector, and thereby a desired base sequence of targeted genomic DNA can be substituted. This substitution is characterized in that it is error-free.

However, because frequency of this homologous recombination in cells derived from mammals is 1 in 1,000,000, which is extremely low, it has been extremely difficult to put the homologous recombination into practical use, especially in terms of applications for medical treatment.

In recent years, discovery of genome editing nucleases has enabled cleavage of DNA double strands at any location in a genome. As a result, it became easy to induce homologous recombination between genomic DNA and a foreign gene to be introduced (refer to, for example, Patent Literature 1, Non Patent Literature 1, and Non Patent Literature 2).

Patent Literature 1 discloses a method in which a genome of a cell of a 1-cell stage embryo is cleaved by Cas9 protein, and a nucleic acid insert (foreign gene to be introduced) is introduced into the cell using a targeting vector including homology arms that respectively hybridize to a 5'-end and a 3'-end of a target sequence, and the nucleic acid insert adjacent to the homology arm.

Non Patent Literature 1 discloses that a normal haemoglobin beta (HBB) gene is introduced into hematopoietic stem cells derived from a β-thalassemia patient with homologous recombination using the CRISPR/Cas9 system in which Cas9 protein and adeno-associated virus (AAV) vectors are combined.

However, even in techniques of recent years, frequency of occurrence of non-homologous recombination is higher than frequency of occurrence of homologous recombination, and the frequency of occurrence of homologous recombination is about one tenth even at the highest level. Accordingly, it is necessary to further increase frequency of homologous recombination when aiming for putting homologous recombination into practical use.

As an attempt to increase frequency of homologous recombination, for example, methods exemplified in Non Patent Literature 3 and Non Patent Literature 4 have been proposed.

Non Patent Literature 2 searches for low molecular weight compounds that inhibit non-homologous end joining or promote homologous recombination in gene transfer with homologous recombination using the CRISPR/Cas9 system. Non Patent Literature 2 discloses that Scr7, L755507, and resveratrol are used as such low molecular weight compounds to promote homologous recombination in porcine fetal fibroblasts.

Non Patent Literature 3 discloses a method in which expression of KU70, DNA ligase IV, and the like is inhibited by RNA interference to reduce frequency of non-homologous recombination, and thereby relatively increasing frequency of homologous recombination.

Non Patent Literature 4 discloses a method of donating single-stranded DNA complementary to a 3'-end of cleaved DNA that is not complementary to sgRNA, which is a 3'-end asymmetrically released from Cas9 before Cas9 dissociates from double-stranded DNA, and thereby increasing frequency of homologous recombination, in genome editing using the CRISPR/Cas9 system.

### [Citation List]

### [Patent Literature]

[Patent Literature 1]
PCT International Publication No. WO2016/081923

### [Non Patent Literature]

[Non Patent Literature 1]
   Nature. 2016 Nov 17; 539(7629): 384-389. CRISPR/Cas9 β-globin gene targeting in human haematopoietic stem cells. Dever D. et al.
[Non Patent Literature 2]
   Sci Rep. 2017; 7: 8943. Small molecules enhance CRISPR/Cas9-mediated homology-directed genome editing in primary cells Guoling Li, et al.
[Non Patent Literature 3]
   Nat Biotechnol. 2015 May; 33(5): 543-548. Increasing the efficiency of homology-directed repair for CRISPR-Cas9-induced precise gene editing in mammalian cells. Chu VT., et al.
[Non Patent Literature 4]
   Nat Biotechnol. 2016; 34: 339-344, Enhancing homology-directed genome editing by catalytically active and inactive CRISPR-Cas9 using asymmetric donor DNA. Richerdson C, et al.

### [Summary of Invention]

### [Technical Problem]

However, even with the methods of Non Patent Literature 3 and Non Patent Literature 4, frequency of homologous recombination is still low, and there is still room for improvement.

Non-homologous end joining and homologous recombination are known as DNA repair mechanisms for DNA double-strand breaks. Non-homologous end joining proceeds in a shorter time than homologous recombination. Accordingly, it is necessary to devise a method of relatively reducing frequency of occurrence of non-homologous end joining in order to increase frequency of homologous recombination. However, when the mechanism of non-homologous end joining itself is inhibited, an ability of cells to repair DNA double-strand breaks is greatly impaired, which poses a significant risk to living organisms (inviability and cancerization). As a result, it is difficult to realize practical use and clinical application of homologous recombination in this direction.

An object of the present invention is to provide a genome editing method, a composition, a cell, a cell preparation, and a method for producing a cell preparation, all of which can increase frequency of homologous recombination without impairing non-homologous end joining, which is an inherent ability of cells.

### [Solution to Problem]

The inventors of the present invention have found that frequency of homologous recombination with respect to non-homologous recombination is extremely high in a case where double-strand breaks of a targeted genomic DNA occur by adopting a short homology arm, which is generally not used for homologous recombination, as a targeting vector at a 5'-end and a 3'-end of foreign DNA. Therefore, the inventors of the present invention have completed the present invention.

That is, the present invention is as follows.
[1] A genome editing method for an isolated cell, the method including introducing foreign DNA into a targeted genome with homologous recombination of at least one of a 5'-end or a 3'-end of the foreign DNA when double-strand breaks of a targeted genomic DNA occur, where the foreign DNA has homology arms, each with a length of less than 500 bp, at the 5'-end and the 3'-end.
[2] The genome editing method according to [1], in which the foreign DNA is introduced into the targeted genome with homologous recombination of both of the 5'-end and the 3'-end of the foreign DNA.
[3] The genome editing method according to [1] or [2], in which the cell is a blood cell or an undifferentiated cell.
[4] The genome editing method according to any one of [1] to [3], in which the cell is a stem cell.
[5] The genome editing method according to any one of [1] to [4], in which the cell is a hematopoietic stem cell.
[6] The genome editing method according to [1], in which the foreign DNA is introduced into the targeted genome with homologous recombination of one of the 5'-end or the 3'-end of the foreign DNA and non-homologous recombination of the other end.
[7] A genome editing method for an isolated cell, the method including introducing foreign DNA into a targeted genome with homologous recombination of both of a 5'-end and a 3'-end of the foreign DNA, where the foreign DNA has homology arms, each with a length of less than 500 bp.
[8] The genome editing method according to [7], in which the cell is a blood cell or an undifferentiated cell.
[9] The genome editing method according to [7] or [8], in which the cell is a stem cell.
[10] The genome editing method according to any one of [7] to [9], in which the cell is a hematopoietic stem cell.
[11] A composition including foreign DNA having homology arms, each with a length of less than 500 bp, at both ends.
[12] The composition according to [11], further including a targeted genomic DNA-cleaving enzyme, or DNA or mRNA encoding the enzyme.
[13] The composition according to [11] or [12], in which the composition is for pharmaceutical use.
[14] The composition according to any one of [11] to [13], in which the composition is used for treatment of severe combined immunodeficiency.
[15] A method for producing a cell preparation for treating severe combined immunodeficiency, the method including, in a cell, introducing foreign DNA into a genome of the cell with homologous recombination of at least one of a 5'-end or a 3'-end of the foreign DNA when double-strand breaks of a targeted genomic DNA occur, where the foreign DNA has homology arms, each with a length of less than 500 bp, and has at least a part of wild-type DNA of the targeted genomic DNA.
[16] The method for producing a cell preparation according to [15], in which the foreign DNA is introduced into the targeted genomic DNA with homologous recombination of both of the 5'-end and the 3'-end of the foreign DNA.
[17] The method for producing a cell preparation according to [15] or [16], in which the cell is a blood cell or an undifferentiated cell.
[18] The method for producing a cell preparation according to any one of [15] to [17], in which the cell is a stem cell.
[19] The method for producing a cell preparation according to any one of [15] to [18], in which the cell is a hematopoietic stem cell.
[20] The method for producing a cell preparation according to [15], in which the foreign DNA is introduced into the targeted genomic DNA with homologous recombination of one of the 5'-end or the 3'-end of the foreign DNA and non-homologous recombination of the other end.
[21] A method for producing a cell preparation for treating severe combined immunodeficiency, the method including, in a cell, introducing foreign DNA into a genome of the cell with homologous recombination of both of a 5'-end or a 3'-end of the foreign DNA, where the foreign DNA has homology arms, each with a length of less than 500 bp, and has at least a part of wild-type DNA of the targeted genomic DNA.
[22] The method for producing a cell preparation according to [21], in which the cell is a blood cell or an undifferentiated cell.
[23] The method for producing a cell preparation according to [21] or [22], in which the cell is a stem cell.
[24] The method for producing a cell preparation according to any one of [21] to [23], in which the cell is a hematopoietic stem cell.
[25] A cell including a fragment derived from foreign DNA at a 5'-end or a 3'-end of a genome insertion site of the foreign DNA in a targeted genome of the cell.
[26] A cell preparation including the cell according to [25].

### [Advantageous Effects of Invention]

According to the present invention, a high frequency of homologous recombination in a targeted genome is realized without impairing non-homologous end joining, which is an inherent ability of cells, as compared with non-homologous recombination.

### [Brief Description of Drawings]

Fig. 1 is a schematic diagram showing repair of an IL2RG gene mutation in a pig with SCID by homologous recombination. Only homologous recombination was detected by a targeting vector having a short homology arm.
Fig. 2 shows results of electrophoresis in which genomic PCR confirmed a state in which an IL2RG gene mutation in hematopoietic stem cells derived from a pig with SCID was repaired only by homologous recombination.
Fig. 3 shows results of electrophoresis in which genomic PCR confirmed a state in which, in a pig with SCID autologously transplanted with hematopoietic stem cells in which a genome was repaired by a genome editing method of the present invention, only blood cells, in which the hematopoietic stem cells were engrafted and thereby a genome was repaired by homologous recombination, were detected.
Fig. 4 is a schematic diagram showing repair of an IL2RG gene mutation in a pig with SCID by causing homologous recombination to occur only at one end of foreign DNA. A 5'-end of the foreign DNA is repaired by non-homologous recombination and a 3'-end thereof is repaired by homologous recombination. In this case, fragments derived from the foreign DNA remain on the 5'-end of a foreign DNA insertion site in the targeted genome.
Fig. 5 shows results of electrophoresis in which genomic PCR confirmed a state in which an IL2RG gene mutation in bone marrow stromal cells derived from a pig with SCID was repaired by homologous recombination for a 5'-end and by non-homologous recombination for a 3'-end.
Fig. 6 shows results of electrophoresis in which genomic PCR confirmed a state in which a GFP gene was inserted into a Rosa26 region of a mouse hematopoietic stem cell genome only with homologous recombination.
Fig. 7A is a fluorescence image in which a genome editing tool of the present invention was microinjected into a fertilized mouse egg. In the fertilized egg in which GFP was detected, at least a 5'-end of the GFP gene was inserted into a β-Actin (Actb) locus with homologous recombination.
Fig. 7B shows results of electrophoresis in which genomic PCR confirmed a state in which the 5'-end of the GFP gene was inserted into the Actb locus of the fertilized mouse egg with homologous recombination, and a 3'-end of the GFP gene was inserted into the Actb locus of the fertilized mouse egg with non-homologous recombination.
Fig. 8 shows results of electrophoresis in which genomic PCR confirmed a state in which a GFP gene was inserted into a hypoxanthine phosphoribosyltransferase (HPRT) locus of a human T-cell leukemia cell line (Jurkat cells) only with homologous recombination.
Fig. 9A shows results of electrophoresis in which genomic PCR confirmed a state in which a GFP gene was inserted into a Lamin B1 (LMNB1) locus of a human embryonic kidney cell line (HEK293T cells) only with homologous recombination.
Fig. 9B is a fluorescence image of HEK293T cells in which a GFP gene was inserted into a LMNB1 locus of the HEK293T cells. A fusion protein of LMNB1 and GFP was expressed by homologous recombination, where this protein was localized in a nuclear envelope. It can be seen that the GFP gene was inserted into the LMNB1 locus only with homologous recombination because GFP was localized in a nuclear envelope in all GFP-positive cells.
Fig. 9C shows results in which efficiency of inserting the GFP gene into the LMNB1 locus in the HEK293T cells was confirmed by flow cytometry.
Fig. 10 shows results of electrophoresis in which genomic PCR confirmed a state in which a GFP gene was inserted into an HPRT locus of bone marrow stromal cells derived from a human only with homologous recombination.
Fig. 11 shows results of electrophoresis in which genomic PCR confirmed a state in which a GFP gene was inserted into an HPRT locus of human iPS cells only with homologous recombination.
Fig. 12 shows results of electrophoresis in which genomic PCR confirmed a state in which gene insertion only with homologous recombination occurred in mouse hematopoietic stem cells even when ZFN and TALEN were used.

### [Description of Embodiments]

### [Genome editing method]

A genome editing method of the present invention is a method including introducing foreign DNA into a targeted genome with homologous recombination of at least one of a 5'-end or a 3'-end of the foreign DNA when double-strand breaks of a targeted genomic DNA occur, where the foreign DNA has homology arms, each with a length of less than 500 bp.

### <First embodiment>

In one embodiment, the present invention provides a genome editing method for a cell, the method including introducing foreign DNA into a genome of the cell with homologous recombination at a 5'-end and a 3'-end of the foreign DNA when double-strand breaks of a targeted genomic DNA occur, where the foreign DNA has homology arms, each with a length of less than 500 bp.

In the present embodiment, first, a double strand of targeted genomic DNA is cleaved at a location, into which foreign DNA is to be introduced, on the targeted genomic DNA. A system used for targeted genomic DNA double-strand breaks is not particularly limited, and examples thereof include a CRISPR-Cas9 system, a Transcription activator-like effector nuclease (TALEN) system, a Zn-finger nuclease system, and the like. A method of introducing these systems into cells is not particularly limited, and a targeted genomic DNA-cleaving enzyme itself may be introduced into cells, or a targeted genomic DNA-cleaving enzyme expression vector may be introduced into cells. A system used for targeted genomic DNA double-strand breaks is introduced into cells simultaneously with foreign DNA, or before or after introduction of foreign DNA.

Examples of methods of introducing the CRISPR-Cas9 system include a method of introducing, into cells, a Cas9 expression vector, and an expression vector encoding guide RNA that induces Cas9 to a location to be cleaved; a method of introducing expressed and purified recombinant Cas9 protein and guide RNA into cells; and the like. Guide RNA may be divided into two, which are tracrRNA and crRNA, or may be sgRNA connected as a single construct.

In the present embodiment, the CRISPR-Cas9 system is preferable as a system used for targeted genomic DNA double-strand breaks.

In the present embodiment, means for introducing foreign gene, a nucleic acid, and a protein into cells is not particularly limited, and it may be any of a method using a viral vector, a non-viral introduction method, or any other known method. Examples of methods using a viral vector include retroviral vectors, lentiviral vectors, adenoviral vectors, Adeno-associated virus (AAV) vectors, herpes virus vectors, Sendai virus vectors, Sindbis virus vectors, and the like. Examples of non-viral introduction methods include a calcium phosphate method, a lipofection method, an electroporation method, a microinjection method, a whisker method, a plasma method, a laser injection method, a particle gun method, an Agrobacterium method, and the like.

In the present embodiment, cells targeted by the genome editing method of the present invention are not particularly limited. Isolated cells are preferable, and examples thereof include animal cells, plant cells, insect cells, fungi such as yeast and mold, bacteria such as Escherichia coli, and the like.

Examples of animal cells include stem cells, germ cells, germline cells, established cells, primary cells, which are derived from animals; and cells induced from stem cells of animals or cells produced from primary cells of animals. The stem cells may also be established cells or primary cells.

The genome editing method of the present invention is not necessarily limited for isolated cells. Targets thereof also include an individual animal itself, or somatic cells and stem cells within the individual animal.

As the cells derived from animals, stem cells are preferable. Stem cells derived from animals are characterized by having (i) self-renewal ability and (ii) pluripotency.

Stem cells of animals are classified into pluripotent stem cells, multipotent stem cells, oligopotent stem cells, and unipotent stem cells according to their different differentiation potentials.

Examples of stem cells of animals include embryonic stem cells such as ES cells and EG cells; ES-like stem cells such as induced pluripotent stem cells (iPS cells); adult stem cells such as fetal stem cells, muse cells, placental stem cells, hematopoietic stem cells, mesenchymal stem cells (dental pulp-derived mesenchymal stem cells, adipose-derived mesenchymal stem cells, bone marrow-derived mesenchymal stem cells, synovium-derived mesenchymal stem cells, and the like), hair follicle stem cells, mammary gland stem cells, neural stem cells, satellite cells, and intestinal epithelial stem cells; and germline stem cells such as GS cells.

The stem cells of animals may be cells obtained by genetic manipulation of stem cells. Examples of such cells include pluripotent stem cells in which immunorejection is suppressed by reorganizing human leukocyte antigens (HLA).

Hematopoietic stem cells are preferable as animal cells.

Examples of germ cells or germline cells include eggs, oocytes, oogonia, sperms, spermatocytes, spermatogonia, sperm stem cells (spermatogonial stem cells), primordial germ cells, and the like. Germ cells or germline cells may be a fertilized egg obtained by fertilization of an egg and a sperm may be used. Furthermore, germ cells or germline cells may be a 2-cell to 8-cell embryo in which a fertilized egg is divided, or a morula to a blastocyst until they undergo implantation.

Established cells of animals is not particularly limited. Examples of established cells of animals include cells derived from ovarian tissue of the Chinese hamster (CHO cells), established cells derived from the kidney of an African green monkey (Vero cells), cells derived from human hepatocellular carcinoma (HepG2 cells), a cell line derived from canine proximal tubular kidney epithelial cells (MDCK cells), a human embryonic kidney line (HEK 293 cells), an established cell line derived from human hepatocellular carcinoma tissue (huGK-14), and the like.

The primary cells of animals are not particularly limited, and the cells may be derived from any of normal tissue or diseased tissue. Examples of primary cells of animals include hair dermal papilla cells, endothelial cell, epithelial cells, epidermal keratinocytes, melanocytes, cardiomyocytes, smooth muscle cells, skeletal muscle cells, skeletal muscle myoblast cells, osteoblasts, chondrocytes, fibroblasts, hepatocytes, nerve cells; immune cells such as regulatory T cells, killer T cells, and gamma delta T cells; and the like.

The cells induced from stem cells of animals may be stem cells or differentiated cells. Examples of cells induced from stem cells of animals include retinal pigment epithelial cells derived from iPS cells, nerve cells derived from iPS cells, immune system cells derived from iPS cells such as killer T cells derived from iPS cells, cardiac progenitor cells derived from iPS cells, hepatocytes derived from iPS cells, and the like.

The cells produced from primary cells of animals are not particularly limited. Typically, the cells produced from primary cells of animals are cells obtained by genetic manipulation of primary cells of animals. Examples of cells produced from primary cells of animals include chimeric antigen receptor (CAR) T cells and the like.

As will be described later in Examples, the inventors of the present invention have found that frequency of occurrence of homologous recombination does not depend on animal species, target loci, transgenes, or types of nucleases cleaving a targeted genome.

In the present embodiment, blood cells or undifferentiated cells are preferable. The undifferentiated cells are more preferably stem cells, and are even more preferably hematopoietic stem cells.

The plant cells are not particularly limited. Examples of plant cells include cells and calluses derived from meristematic tissues or seeds of plants, and the like. Calluses may be any of calluses induced from fragments of plant tissue, wound-induced calluses, bacteria-induced calluses, calluses formed by interspecific hybridization, and cultured cells. Typically, the cells and calluses derived from meristematic tissues or seeds of plants are characterized by expressing at least one of pluripotency markers such as PLT1, PLT5, LBD16, LBD17, LBD18, LBD29, ARR1, ARR21, ESR1, ESR2, WIND1, WIND2, WIND3, WIND4, LEC1, LEC2, AGL15, BBM, RKD1, RKD2, and WUS. A genome editing method for plant cells will be described later.

Next, in the present embodiment, a targeting vector having homology arms, each with a length of less than 500 bp, at both ends of foreign DNA is introduced into a cell. A homology arm refers to DNA which is provided at a 5'-end and a 3'-end of foreign DNA to be inserted and has a sequence homologous to a site of a genome on which insertion is performed.

An upper limit value of a length of the homology arm is less than 500 bp, is preferably equal to or less than 300 bp, is more preferably equal to or less than 100 bp, and is particularly preferably equal to or less than 50 bp, and it may be equal to or less than 10 bp. An introduced homology arm contributes to homologous recombination at both sides of the 5'-end and the 3'-end.

A lower limit value of a length of the homology arm is preferably equal to or more than 5 bp, and is more preferably equal to or more than 10 bp.

A length of the homology arm is preferably 5 to 499 bp, more preferably 5 to 300 bp, even more preferably 5 to 100 bp, particularly preferably 5 to 50 bp, and most preferably 10 to 50 bp.

A length of the foreign DNA is not particularly limited as long as it can be inserted into the genome. Examples of lengths of the foreign DNA include 50 bp to 10 kbp, 100 to 5 kbp, 100 to 1 kbp, 100 to 500 bp, and the like. Examples of foreign DNA include wild-type DNA having a target sequence, DNA having a codon-optimized sequence, tagged foreign DNA, a promoter sequence, a transcription termination sequence, a functional gene sequence, a fluorescent protein marker gene sequence, a drug-selection gene sequence, a multiple cloning site sequence, and a combination thereof, and the like.

In the present embodiment, the type of targeting vector used is not particularly limited, and it is possible to use conventionally known vectors such as a plasmid vector and a viral vector.

Examples of viral vectors include retroviral vectors, lentiviral vectors, adenoviral vectors, Adeno-associated virus (AAV) vectors, herpes virus vectors, Sendai virus vectors, Sindbis virus vectors, and the like.

Examples of targeting vectors include various vectors for genome editing using a CRISR/Cas9 system, and examples thereof include a targeting vector using a homology-independent targeted integration (HITI) system.

In general, it is thought that homologous recombination is performed more efficiently when a length of a homology arm in a gene targeting vector for the homologous recombination becomes longer from the viewpoint that it increases a proportion of a homologous region. However, the inventors of the present invention have found that it is possible to obtain a completely unexpected result which is occurrence of homologous recombination at a significantly higher frequency than non-homologous recombination in foreign DNA having short homology arms at both of a 5'-end and a 3'-end in a case where double-strand breaks of a targeted genomic DNA occur.

By the genome editing method of the present embodiment, it is possible to realize an extremely high frequency of homologous recombination at both sides of a 5'-end and a 3'-end of foreign DNA with respect to non-homologous recombination. Specifically, in the method of the present invention, by using a targeting vector in which short homology arms, each with a length of less than 500 bp, are used, it is possible to realize a significantly higher frequency of homologous recombination than that of non-homologous recombination while still maintaining an ability of cells to repair DNA double-strand breaks and without inhibiting the mechanism of non-homologous end joining, which is an inherent ability of cells.

Accordingly, the genome editing method of the present embodiment expands possibilities of applications of the genome editing techniques for medical treatment and industry. The present invention can produce blood cells that can be used to treat individuals with diseases caused by gene mutations. The diseases caused by gene mutations are not particularly limited, and for example, a disease may be any of congenital immunodeficiency disorders (such as, in addition to X-SCID of Examples, adenosine deaminase [ADA] deficiency, chronic granulomatous disease, X-linked agammaglobulinemia [XLA], ZAP-70 deficiency, hyper IgM Syndrome, IgA deficiency, IgG subclass deficiency, Bloom syndrome, Wiskott-Aldrich syndrome, Ataxia-telangiectasia, and DiGeorge syndrome), Fanconi anemia, thalassemia, sickle cell anemia, leukodystrophy, hemophilia, mucopolysaccharidosis, or the like. Since the present invention realizes a higher frequency of homologous recombination than non-homologous recombination, the present invention is extremely useful for treatment of various diseases, which are difficult to be treated with non-homologous recombination but are expected to be treated with homologous recombination, such as diseases with mutations in giant genes (muscular dystrophy and the like) and diseases with long gene mutations (triplet repeat disorders such as Huntington's disease, and the like). The adaptation of the present invention is not necessarily limited to treatment of the diseases caused by gene mutations. For example, the present invention can be widely utilized for functional modification of mesenchymal stem cells and T cells. Examples thereof include modification of an HLA locus of mesenchymal stem cells and CAR-T cells, and the like. These genome-modified cells created by the present invention can be provided for treatment of various cancers, leukemia, hematopoietic disorders, myelodysplastic syndromes, ischemic diseases such as myocardial infarction, cerebral infarction, and arteriosclerosis obliterans, Buerger's disease, peripheral disease, critical limb ischemia, pulmonary hypertension, autoimmune disease, lupus nephritis, Crohn's disease, corneal disease, corneal disorders, glaucoma, optic nerve disorders, retinitis pigmentosa, macular dystrophy, and the like. The applications of the present invention are not limited to medical treatment. There are possibilities of applications of the genome editing method for animal cells according to the present invention for, for example, creation of beef cattle which have a high unsaturated fatty acid content and are excellent in taste, tuna in which a proportion of otoro (fattiest portion) is increased, and the like.

### <Second embodiment>

In one embodiment, the present invention provides a genome editing method for a cell, the method including introducing foreign DNA into a genome of the cell with homologous recombination at one end of a 5'-end and a 3'-end of the foreign DNA and non-homologous recombination at the other end when double-strand breaks of a targeted genomic DNA occur, where the foreign DNA has homology arms, each with a length of less than 500 bp.

Examples of the present embodiment include fertilized mouse eggs, pig bone marrow stromal cells, and the like.

As a means for increasing efficiency of genome editing of the present embodiment, a length of one homology arm in the foreign DNA is preferably two times or more, more preferably three times or more, even more preferably four times or more, and particularly preferably five times or more a length of the other homology arm.

A length of the short homology arm is preferably equal to or less than 50 bp, more preferably equal to or less than 30 bp, and particularly preferably equal to or less than 10 bp, and it may be equal to or less than 0 bp. A length of the long homology arm is preferably equal to or more than 30 bp, more preferably equal to or more than 40 bp, and particularly preferably equal to or more than 50 bp.

An introduced short homology arm contributes to non-homologous recombination, and an introduced long homology arm contributes to homologous recombination. In the present embodiment, non-homologous recombination means non-homologous end joining.

A length of the foreign DNA is not particularly limited as long as it can be inserted into the genome, and examples thereof include 100 bp to 10 kbp.

The mechanism of occurrence of lateral homologous recombination in the genome editing method of the present embodiment is unclear, but it is presumed as follows. Homologous recombination is less likely to occur at a short homology arm than a long homology arm, and a location at which a genomic DNA double-strand break occurs is connected with the short homology arm by the mechanism of non-homologous recombination (non-homologous end joining). When one end of a transgene is connected to genomic DNA in this manner, this connecting site becomes a fulcrum, causing the long homology arm at the other end to be located near a homologous sequence in a genome, and thereby homologous recombination becomes likely to occur. That is, it is thought that one end of foreign DNA is first connected by non-homologous recombination, and then the foreign DNA is inserted into the genome by the homologous recombination at the other end side.

### <Third embodiment>

In one embodiment, the present invention provides a genome editing method for an isolated cell, the method including introducing foreign DNA into a targeted genome with homologous recombination of both of a 5'-end and a 3'-end of the foreign dna, where the foreign dna has homology arms, each with a length of less than 500 bp.

In the present embodiment, a vector that causes homologous recombination without targeted genomic DNA double-strand breaks is used as a targeting vector. Examples of such a targeting vector include an AAV vector containing single-stranded DNA.

It is the same as that of the first embodiment except that it does not cause targeted genomic DNA double-strand breaks.

### [Composition]

A composition of the present invention contains foreign DNA having homology arms, each with a length of less than 500 bp, at both ends.

The composition of the present invention may contain a targeted genomic DNA-cleaving enzyme, or DNA or mRNA encoding the enzyme, or instead of being a DNA-cleaving enzyme, it may be a nickase that introduces a nick in one side of double-stranded DNA, or a helicase that separates double-stranded DNA into a single strand.

In the present invention, examples of targeted genomic DNA-cleaving enzymes include Cas9, Transcription activator-like effector nuclease (TALEN), Zn-finger nuclease, and the like. In addition, examples of DNA or mRNA encoding the enzyme include DNA or mRNA encoding these proteins.

When Cas9 is used as the targeted genomic DNA-cleaving enzyme, the composition preferably contains guide RNA that induces Cas9. In addition, the composition may also contain an expression vector encoding guide RNA.

In the present invention, a length of each of the homology arms provided at the both ends of the foreign DNA is less than 500 bp, and is preferably equal to or less than 300 bp, meaning that the homology arms are the same as those in the above-described [Genome editing method].

In the present invention, the foreign DNA is the same as that in the above-described [Genome editing method], and the composition of the present invention may contain a targeting vector containing the foreign DNA. The targeting vector is the same as that in the above-described [Genome editing method].

In the present invention, the above-described foreign DNA may be contained in one kind of vector, or may be contained in a plurality of kinds of vectors. The vector is not particularly limited, and it is the same as that in the above-described [Genome editing method].

The composition of the present invention is preferably for pharmaceutical use, and it more preferably contains a pharmaceutically acceptable carrier. The composition for pharmaceutical use of the present embodiment is administered orally in the form of, for example, tablets, coated tablets, pills, powders, granules, capsules, liquids, suspensions, emulsions, and the like, or it is administered parenterally in the form of injections, suppositories, external preparations for skin, and the like.

As the pharmaceutically acceptable carrier, carriers generally used for formulation of pharmaceutical compositions can be used without particular limitation. More specific examples thereof include binders such as gelatin, corn starch, gum tragacanth, and gum arabic; excipients such as starch and crystalline cellulose; swelling agents such as alginic acid; injectable solvents such as water, ethanol, and glycerin; adhesives such as rubber adhesives and silicone adhesives; and the like. One kind of pharmaceutically acceptable carrier is used alone, or two or more kinds thereof are mixed and used.

The composition of the present invention may further contain additives. Examples of additives include lubricants such as calcium stearate and magnesium stearate; sweeteners such as sucrose, lactose, saccharin, and maltitol; flavoring agents such as peppermint and a Gaultheria adenothrix oil; stabilizers such as benzyl alcohol and phenol; buffering agents such as phosphate and sodium acetate; solubilizing agents such as benzyl benzoate and benzyl alcohol; antioxidants; preservatives; and the like.

One kind of additive is used alone, or two or more kinds thereof are mixed and used.

The composition of the present invention is preferably used for treatment of severe combined immunodeficiency. Regarding severe combined immunodeficiency (SCID), the most common form of this disorder is the X-linked type, which is caused by mutations in an IL-2 receptor γ gene (IL2RG).

In the present invention, foreign DNA contained in the composition for treating X-linked severe combined immunodeficiency preferably contains at least a part of a wild-type IL-2 receptor γ gene. In addition, when Cas9 is used as a targeted genomic DNA-cleaving enzyme, it preferably contains guide RNA that hybridizes to the IL-2 receptor γ gene in a targeted genome, or an expression vector encoding the guide RNA.

It becomes possible to provide the genome editing method of the present invention by using the composition of the present invention.

### [Gene treatment method]

A gene treatment method of the present invention is a method including administering a pharmaceutical composition to a target, in which the pharmaceutical composition contains an enzyme cleaving targeted genomic DNA having mutations or DNA or mRNA encoding the enzyme, and contains foreign DNA having homology arms, each with a length of less than 500 bp, at both ends, and having at least a part of wild-type DNA of the targeted genomic DNA.

In the present invention, a mutation is caused by deletion, substitution, insertion of an arbitrary sequence, or the like in exons and introns of targeted genomic DNA, or in an expression control region of the targeted genomic DNA.

In the present invention, an administration method is not particularly limited, and it may be appropriately determined according to symptoms, body weight, age, gender, and the like of a patient. For example, tablets, coated tablets, pills, powders, granules, capsules, liquids, suspensions, emulsions, and the like are orally administered. In addition, injections are administered intravenously alone or in combination with ordinary replacement fluids such as glucose and amino acids, and if necessary, injections are further administered intramedullary, intraarterially, intramuscularly, intradermally, subcutaneously, or intraperitoneally.

In the present invention, a dosage of the pharmaceutical composition varies depending on symptoms, body weight, age, gender, and the like of a patient, and thus it cannot be comprehensively determined. However, in a case of oral administration, it is sufficient for an active ingredient to be administered by, for example, 1 µg to 10 g per day, or for example, 0.01 to 2,000 mg per day. In addition, in a case of an injection, it is sufficient for an active ingredient to be administered by, for example, 0.1 µg to 1 g per day, or for example, 0.001 to 200 mg per day.

### [Cell]

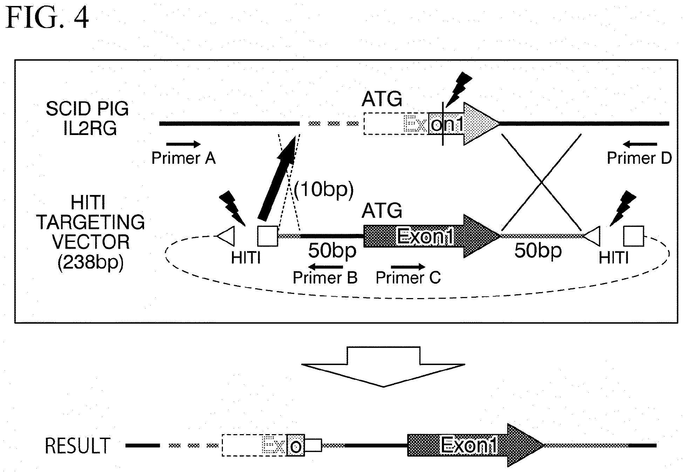

In the second embodiment, a cell of the present invention is characterized in that a fragment derived from foreign DNA remains at a 5'-end or a 3'-end of a genome insertion site of the foreign DNA in a targeted genome of the cell. According to the genome editing method of the second embodiment described above, through non-homologous recombination, one end of foreign DNA is first connected to one end of targeted genomic DNA generated by double-strand breaks. Accordingly, the cell of the present invention includes a fragment derived from foreign DNA at a 5'-end or a 3'-end of a genome insertion site of the foreign DNA. As shown in results of Fig. 4, the cell includes a fragment derived from foreign DNA at a 5'-end of a genome insertion site of the foreign DNA in a case where non-homologous recombination occurs at the 5'-end of targeted genomic DNA. The cell includes a fragment derived from foreign DNA at a 3'-end of a genome insertion site of the foreign DNA in a case where non-homologous recombination occurs at the 3'-end of targeted genomic DNA.

### [Cell preparation]

A cell preparation of the present invention contains, for example, cells in which targeted genomic DNA having a mutation is edited in a wild-type by using the genome editing method of the second embodiment described above. Furthermore, as described in the above-described [Cell], the cell preparation of the present invention contains the cell in which a fragment derived from foreign DNA remains at a 5'-end or a 3'-end of a genome insertion site of the foreign DNA in a targeted genome of the cell.

### [Method for producing cell preparation]

A method for producing a cell preparation of the present invention is a method for producing a cell preparation for treating severe combined immunodeficiency, the method including, in a cell, introducing foreign DNA into a genome of the cell with homologous recombination of at least one of a 5'-end or a 3'-end of the foreign DNA when double-strand breaks of a targeted genomic DNA occur, where the foreign DNA has homology arms, each with a length of less than 500 bp, and has at least a part of wild-type DNA of the targeted genomic DNA.

In one embodiment, the present invention provides a method for producing a cell preparation for treating severe combined immunodeficiency, the method including, in a cell, introducing foreign DNA into a genome of the cell with homologous recombination of both of a 5'-end or a 3'-end of the foreign DNA when double-strand breaks of a targeted genomic DNA occur, where the foreign DNA has homology arms, each with a length of less than 500 bp, and has at least a part of wild-type DNA of the targeted genomic DNA.

In one embodiment, the present invention provides a method for producing a cell preparation for treating severe combined immunodeficiency, the method including, in a cell, introducing foreign DNA into a genome of the cell with homologous recombination of one of a 5'-end or a 3'-end of the foreign DNA and non-homologous recombination of the other end when double-strand breaks of a targeted genomic DNA occur, where the foreign DNA has homology arms, each with a length of less than 500 bp, and has at least a part of wild-type DNA of the targeted genomic DNA.

In one embodiment, the present invention provides a method for producing a cell preparation for treating severe combined immunodeficiency, the method including, in a cell, introducing foreign DNA into a genome of the cell with homologous recombination of both of a 5'-end or a 3'-end of the foreign DNA, where the foreign DNA has homology arms, each with a length of less than 500 bp, and has at least a part of wild-type DNA of the targeted genomic DNA.

Cells serving as a host of the cell preparation are not particularly limited. Examples thereof include the same cells as those in the above-described [Genome editing method], and cells may be cells derived from bone marrow taken out from a human body.

Genome-edited cells are proliferated in vitro in this manner and then administered to a patient as a cell preparation by intravenous injection or the like.

By using the method for producing a cell preparation of the present invention, a high frequency of homologous recombination in a targeted genome is realized as compared with non-homologous recombination. As a result, the cell preparation can be efficiently produced.

According to the method for producing a cell preparation of the present invention, it is possible to provide a cell preparation for error-free repair of targeted genomic DNA having mutations in cells derived from bone marrow of a patient with severe combined immunodeficiency to a healthy base sequence through homologous recombination, and complete curing of severe combined immunodeficiency.

### [Genome editing method for plant cells]

In one embodiment, the present invention provides a genome editing method for plant cells, the method including introducing foreign DNA into a genome of the cell with homologous recombination at a 5'-end and a 3'-end of the foreign DNA when double-strand breaks of a targeted genomic DNA occur, where the foreign DNA has homology arms, each with a length of less than 500 bp.

In the present embodiment, systems used for targeted genomic DNA double-strand breaks are not particularly limited, and systems are the same as those in the above-described [Genome editing method]. A method of introducing these systems into cells is also not particularly limited, and it is the same as that in the above-described [Genome editing method].

In the present embodiment, a means for introducing a foreign gene into cells is also not particularly limited, and it is the same as that in the above-described [Genome editing method].

Plant cells are not particularly limited, and plant cells are the same as those in the above-described [Genome editing method]. Plant cells are preferably cells and calluses derived from meristematic tissues or seeds of plants, and the like.

An upper limit value of a length of the homology arm is less than 500 bp, is preferably equal to or less than 300 bp, is more preferably equal to or less than 100 bp, and is particularly preferably equal to or less than 50 bp, and it may be equal to or less than 10 bp. A lower limit value of a length of the homology arm is preferably equal to or more than 5 bp, and is more preferably equal to or more than 10 bp. A length of the homology arm is not particularly limited in the range between the upper limit value and the lower limit value, and a length thereof is the same as that in the above-described [Genome editing method].

A length of the foreign DNA is not particularly limited as long as it can be inserted into the genome, and a length thereof is the same as that in the above-described [Genome editing method].

In the present embodiment, the type of targeting vector used is not particularly limited, and it is the same as that in the above-described [Genome editing method].

Genome editing in plant cells may be performed by an in vitro culture system or may be performed in planta. In a genome editing method by an in vitro culture system, introduction of foreign genes, nucleic acids, and proteins into cells is performed on calluses or fragments of tissue using known methods such as an Agrobacterium method, a particle gun method, and a whisker method. In a genome editing method performed in planta, introduction of foreign genes, nucleic acids, and proteins into cells is performed on shoot apices of exposed immature embryos and mature embryos using a known method. For cereals such as wheat, rice, corn, and soybean, a method of introducing into ripe seed embryos by using a particle gun method is preferable from the viewpoint of efficiency of introduction into a plant body. In addition, the method is applied to cereals such as barley and potatoes; vegetables such as tomatoes and cabbages; flowers such as carnations, roses, sweet pea, and chrysanthemums; and the like.

The genome editing method for plant cells of the present embodiment expands possibilities of applications of the genome editing techniques for the field of agriculture. Specific examples thereof include creation of sake rice which has a low carbohydrate content and is less likely to cause a hangover.

### [Examples]

Hereinafter, the present invention will be described in more detail with reference to experimental examples, but the present invention is not limited to these examples.

### <Experimental Example 1>

### [Construction of donor plasmid]

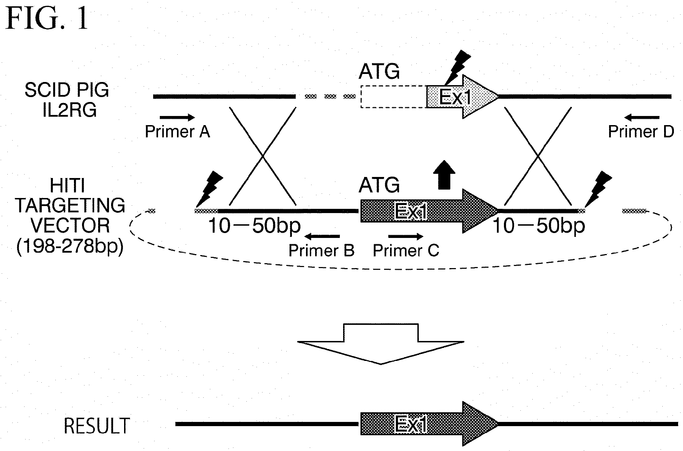

A donor plasmid was produced using a donor plasmid (HITI targeting vector) for repairing mutations from a 5'-control region to the middle of exon 1 (85bp and 1bp deficiencies, 2bp and 1bp base substitutions) in an interleukin-2 receptor gamma gene (hereinafter also referred to as IL2RG) in a genome of hematopoietic stem cells derived from an SCID pig model described in Watanabe M et al., PLoS One., 2013 Oct 9; 8(10): e76478. A structure of the donor plasmid is shown in Fig. 1. The donor plasmid has a structure in which homology arms of the following combination are added to 155 bp foreign DNA containing a mutation site.
(a) A homology arm with 10 bp at a 5'-end and a homology arm with 50 bp at a 3'-end
(b) Homology arms, each with 50 bp, at both 5'-end and 3'-end
(c) A homology arm with 50 bp at a 5'-end and a homology arm with 10 bp at a 3'-end
(d) Homology arms, each with 10 bp, at both 5'-end and 3'-end

A base sequence containing the homology arm with 10 bp at the 5'-end is shown below.

### 5'-GGCCCAGGTT-3' (SEQ ID NO: 1)

A base sequence containing the homology arm with 50 bp at the 5'-end is shown below.

5'–CAAAAGGAAATGTGTGGGTGGGGAGGGGTAGTGGGTAAGGGGCC
CAGGTT–3' (SEQ ID NO: 2)

A base sequence of foreign DNA is shown below. Lowercase letters indicate a 5'-control region lacking in a pig with SCID, and capital letters indicate a base sequence of codon-optimized Ex1 (exon 1).

5'–cctgacacagtctacacccaggaaacaaggagtaagcgccATGCTCAAACCCCCCCTCCC

CGTCAAGTCTCTCCTCTTCCTCCAGCTCCCTCTGCTCGGCGTCGGCCTCAATCC

TAAGGTCCTCACCCACAGCGGCAACGAGGACATCACCGCTG–3' (SEQ ID NO:

3)

A base sequence containing the homology arm with 50 bp at the 3'-end is shown below.
5'-GTGGGAAACTGGGACGTTGGGGGTAGGGTTGGTGAGCCGGGGGA GGCTGG-3' (SEQ ID NO: 4)A base sequence containing the homology arm with 10 bp at the 3'-end is shown below.
5'-GTGGGAAACT-3' (SEQ ID NO: 5)

An HITI base sequence added to both ends of the homology arms is shown below.
5'-CCTTCGGGTTCAGTCCCACCCCA-3' (SEQ ID NO: 6)

### [Introduction of wild-type IL2RG-Ex1 gene into IL2RG-deficient pig hematopoietic stem cells using CRISPR-Cas9]

Cas9 protein, crRNA shown in SEQ ID NO: 7

(5'-UGGGGUGGGACUGAACCCGAGUUUUAGAGCUAUGCU-3'), tracrRNA (Alt-R (registered trademark) CRISPR-Cas9 tracrRNA, Catalog No. 1072534, manufactured by Integrated DNA Technologies, Inc.), and the above-mentioned donor plasmid were introduced into IL2RG-deficient pig hematopoietic stem cells by electroporation. As the pig hematopoietic stem cells, various (CD3, CD16, and CD45RA) differentiation marker negative cells (Lin-) in the pig bone marrow were used.

### [Confirmation of insertion by genomic PCR]

Genomic DNA was purified from the cells 7 days after the electroporation, and foreign DNA insertion was confirmed by PCR. As primers used for confirmation of insertion, a combination of A and B and a combination of C and D shown in Fig. 1 were used. When homologous recombination at the 5'-end occurred, 335 bp DNA was amplified by PCR using the combination of primers A and B. When homologous recombination at the 3'-end occurred, 197 bp DNA was amplified by PCR using the combination of primers C and D.

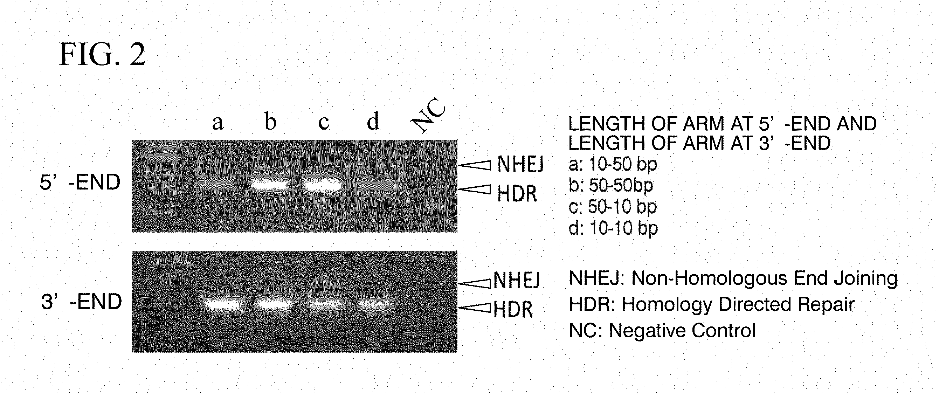

Fig. 2 shows results of electrophoresis of amplified PCR products. As shown in Fig. 2, only bands of a size indicating that homologous recombination had occurred at the 5'-end and the 3'-end were recognized. In Fig. 2, non-homologous end joining (NHEJ) shows a band size corresponding to non-homologous recombination, and homology directed repair (HDR) shows a band size corresponding to homologous recombination.

### [Confirmation of recombination method from sequence]

A TA-cloned PCR product was introduced into Escherichia coli, and each colony formed was sequenced.

A combinations of homology arms used are as follows.
(a) A homology arm with 10 bp at a 5'-end and a homology arm with 50 bp at a 3'-end
(b) Homology arms, each with 50 bp, at both 5'-end and 3'-end
(c) A homology arm with 50 bp at a 5'-end and a homology arm with 10 bp at a 3'-end
(d) Homology arms, each with 10 bp, at both 5'-end and 3'-end

At the 5'-end, homologous recombination occurred in all clones of (a) 4 clones, (b) 8 clones, (c) 7 clones, and (d) 6 clones in which recombination had occurred, and at the 3'-end, homologous recombination occurred in all clones of (a) 8 clones, (b) 7 clones, (c) 8 clones, and (d) 10 clones in which recombination had occurred. Therefore, it was confirmed that homologous recombination occurred at an extremely high frequency (100%) at both of the 5'-end and the 3'-end. In addition, it was confirmed that a mutant sequence repaired by such homologous recombination was a sequence encoding exon 1 of wild-type IL2RG (error-free repair).

### [Transplantation of IL2RG-deficient pig hematopoietic stem cells into which IL2RG-Ex1 gene was introduced into SCID pig and detection thereof]

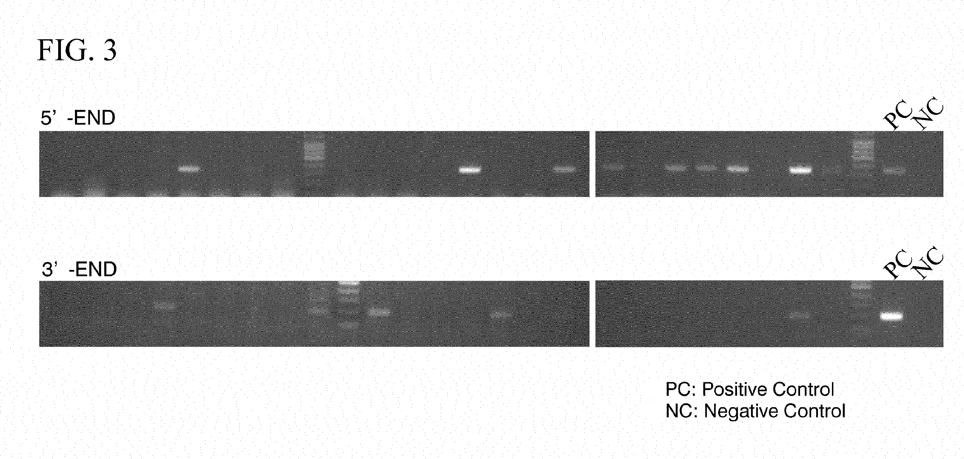

The IL2RG-deficient pig hematopoietic stem cells that had undergone the genome repair were autologously transplanted into an IL2RG-deficient pig of the same individual. Four weeks after the transplantation, cells derived from peripheral blood of the individual were collected, and the presence or absence of cells in which a genome was repaired by homologous recombination was confirmed by PCR analysis. As a result, only a band corresponding to homologous recombination was detected, and a band corresponding to non-homologous recombination was not detected (Fig. 3). Furthermore, when the sequence analysis of the obtained PCR product was performed, it was confirmed that a gene mutation of the SCID pig was error-freely repaired to a healthy base sequence by homologous recombination. This indicates that genetic abnormality of the pig was repaired exclusively by homologous recombination of the targeting vector.

### <Experimental Example 2>

### [Construction of donor plasmid]

A donor plasmid (targeting vector), which is shown Fig. 4 and is for repairing IL2RG in a genome of bone marrow stromal cells derived from an SCID pig model described in Watanabe M et al., PLoS One., 2013 Oct 9; 8(10): e76478, was produced.

A base sequence of a homology arm (10 bp) at a 5'-end is the same as that of SEQ ID NO: 1.

A base sequence of Ex1 (exon 1) is the same as that of SEQ ID NO: 3.

A base sequence of a homology arm (50 bp) at a 3'-end is the same as that of SEQ ID NO: 4.

An HITI base sequence added to both ends of the homology arms is the same as that of SEQ ID NO: 6.

### [Insertion of wild-type IL2RG-Exl gene into IL2RG-deficient pig cells using CRISPR-Cas9]

Cas9 protein, crRNA shown in SEQ ID NO: 7, tracrRNA, and the above-described donor plasmid were introduced by electroporation. As bone marrow stromal cells derived from a pig, adherent cells obtained by liquid-culturing pig bone marrow mononuclear cells were used.

### [Confirmation of insertion by genomic PCR]

Genomic DNA was purified from the cells 3 days after the electroporation, and DNA insertion was confirmed by PCR. As primers used for confirmation of insertion, a combination of A and B and a combination of C and D shown in Fig. 4 were used. In PCR using the combination of primers A and B, 389 bp DNA was amplified when non-homologous recombination occurred. In PCR using the combination of primers C and D, 399 bp DNA was amplified when non-homologous recombination occurred, and 301 bp DNA was amplified when homologous recombination occurred.

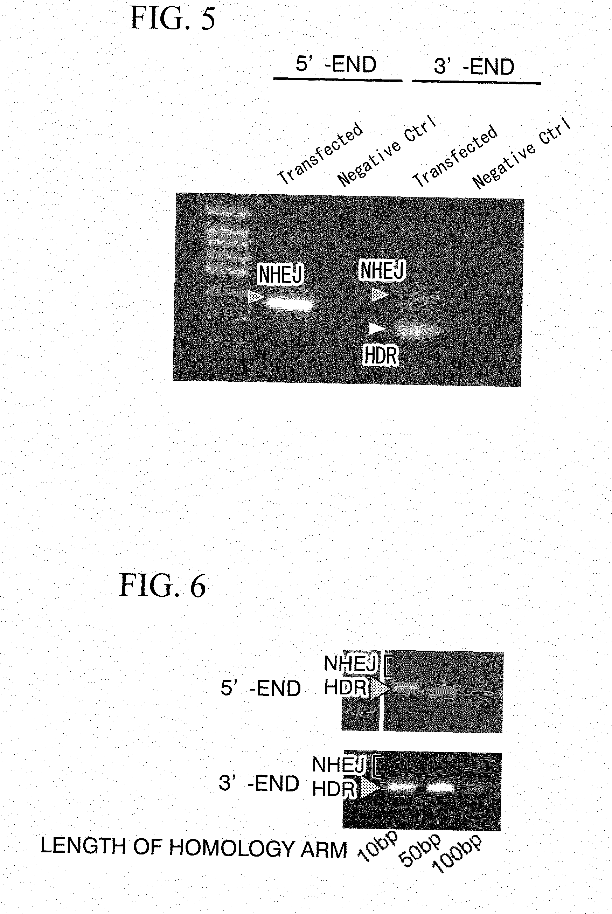

Fig. 5 shows results of electrophoresis of a PCR product. As shown in Fig. 5, at the 5'-end, a band of a size corresponding to non-homologous recombination was recognized, and at the 3'-end, bands of a size corresponding to homologous recombination and non-homologous recombination were recognized. In Fig. 5, NHEJ indicates a band size corresponding to non-homologous recombination, and HDR indicates a band size corresponding to homologous recombination. That is, in bone marrow stromal cells derived from a pig, non-homologous recombination occurred at the 5'-end of foreign DNA, and lateral homologous recombination called homologous recombination occurred at the 3'-end of the foreign DNA. This indicates that non-homologous recombination occurred at the 5'-end (10 bp) on the shorter homology arm, and homologous recombination occurred at the 3'-end (50 bp) on the longer homology arm.

### [Confirmation of recombination method from sequence]

A TA-cloned PCR product was introduced into Escherichia coli, and each colony formed was sequenced. It was confirmed that non-homologous recombination occurred in all clones of 5 clones at the 5'-end, and homologous recombination occurred at a high frequency of 6 clones out of 7 clones at the 3'-end.

### <Experimental Example 3>

A donor plasmid containing homology arm, each with 10 bp, at both ends, a donor plasmid containing homology arm, each with 50 bp, at both ends, and a donor plasmid containing homology arms, each with 100 bp, at both ends were produced for knocking in multicloning site (MCS), GFP, and blasticidin S deaminase (BSR) genes at a Rosa26 region of a mouse hematopoietic stem cell genome and a β-Actin (Actb) locus. As mouse hematopoietic stem cells, various (CD5, CD45R, CD11b, Gr-1, 7-4, and Ter-119) differentiation marker negative cells (Lin-) in mouse bone marrow were used.

Abase sequence of a homology arm with 10 bp at a 5'-end targeting the Rosa26 region is shown below.
5'-TGCAACTCCA-3' (SEQ ID NO: 8)

A base sequence of a homology arm with 50 bp at a 5'-end targeting the Rosa26 region is shown below.

5'–TGGGCCTGGGAGAATCCCTTCCCCCTCTTCCCTCGTGATCTGCAAC

TCCA–3' (SEQ ID NO: 9)

A base sequence of a homology arm with 100 bp at a 5'-end targeting the Rosa26 region is shown below.

5'-AATACCTTTCTGGGAGTTCTCTGCTGCCTCCTGGCTTCTGAGGACC
GCCCTGGGCCTGGGAGAATCCCTTCCCCCTCTTCCCTCGTGATCTGCAACTCC
A-3' (SEQ ID NO: 10)

A base sequence of a homology arm with 10 bp at a 3'-end targeting the Rosa26 region is shown below.
5'-ACAGGTGTAA-3' (SEQ ID NO: 11)

A base sequence of a homology arm with 50 bp at a 3'-end targeting the Rosa26 region is shown below.

5'-ACAGGTGTAAAATTGGAGGGACAAGACTTCCCACAGATTTTCGGT

TTTGT–3' (SEQ ID NO: 12)

A base sequence of a homology arm with 100 bp at a 3'-end targeting the Rosa26 region is shown below.

5'–ACAGGTGTAAAATTGGAGGGACAAGACTTCCCACAGATTTTCGGT
TTTGTCGGGAAGTTTTTAATAGGGGCAAATAAGGAAAATGGGAGGATAGGTA
GT–3' (SEQ ID NO: 13)

A base sequence of foreign DNA containing a GFP gene is shown below.

5'–actagttctagcatctgtagggcgcagtagtccagggtttccttgatgatgtcatacttatcctgtcccttttttttcc
acagctcgcggttgaggacaaactcttcgcgcatgcggatccggtaccATGGTGAGCAAGGGCGAGGAG
CTGTTCACCGGGGTGGTGCCCATCCTGGTCGAGCTGGACGGCGACGTAAACG
GCCACAAGTTCAGCGTGTCCGGCGAGGGCGAGGGCGATGCCACCTACGGCAA
GCTGACCCTGAAGTTCATCTGCACCACCGGCAAGCTGCCCGTGCCCTGGCCC
ACCCTCGTGACCACCCTGACCTACGGCGTGCAGTGCTTCAGCCGCTACCCCGA
CCACATGAAGCAGCACGACTTCTTCAAGTCCGCCATGCCCGAAGGCTACGTCC
AGGAGCGCACCATCTTCTTCAAGGACGACGGCAACTACAAGACCCGCGCCGA
GGTGAAGTTCGAGGGCGACACCCTGGTGAACCGCATCGAGCTGAAGGGCATC
GACTTCAAGGAGGACGGCAACATCCTGGGGCACAAGCTGGAGTACAACTACA
ACAGCCACAACGTCTATATCATGGCCGACAAGCAGAAGAACGGCATCAAGGT
GAACTTCAAGATCCGCCACAACATCGAGGACGGCAGCGTGCAGCTCGCCGAC
CACTACCAGCAGAACACCCCCATCGGCGACGGCCCCGTGCTGCTGCCCGACA
ACCACTACCTGAGCACCCAGTCCGCCCTGAGCAAAGACCCCAACGAGAAGCG
CGATCACATGGTCCTGCTGGAGTTCGTGACCGCCGCCGGGATCACTCTCGGCA
TGGACGAGCTGTACAAGTAAgaattc–3' (SEQ ID NO: 14)

An HITI base sequence added to both ends of the homology arms when targeting the Rosa26 region is shown below.
5'-CCATCTTCTAGAAAGACTGGAGT-3' (SEQ ID NO: 15)

In the same manner as in Experimental Examples 1 and 2, Cas9 protein, crRNA shown in SEQ ID NO: 16 (5'-ACUCCAGUCUUUCUAGAAGAGUUUUAGAGCUAUGCU-3') targeting the Rosa26 region, tracrRNA, and the above-described donor plasmid were introduced into mouse hematopoietic stem cells by electroporation.

Genomic DNA was purified from the cells 3 days after the electroporation, and DNA insertion was confirmed by PCR. As shown in Fig. 6, only bands of a size corresponding to homologous recombination at the 5'-end and the 3'-end were recognized. That is, the GFP gene was inserted into the Rosa26 region of the mouse hematopoietic stem cell genome only with homologous recombination.

### <Experimental Example 4>

A donor plasmid containing homology arms, each with 100 bp, at both ends was produced for knocking in a GFP gene at an Actb locus of fertilized mouse eggs. Because a stop codon was provided on the outside (5'-end side) of the homology arm at the 5'-end, GFP was expressed when homologous recombination occurred at the 5'-end.

A base sequence containing the homology arm with 100 bp at a 5'-end is shown below.

5'-GGATCGGTGGCTCCATCCTGGCCTCACTGTCCACCTTCCAGCAGAT

GTGGATCAGCAAGCAGGAGTACGATGAGTCCGGCCCCTCCATCGTGCACCGC

AA–3' (SEQ ID NO: 17)

A base sequence containing the homology arm with 100 bp at a 3'-end is shown below.

5'–GGACTGTTACTGAGCTGCGTTTTACACCCTTTCTTTGACAAAACCT
AACTTGCGCAGAAAAAAAAAAAAATAAGAGACAACATTGGCATGGCTTTGTTT
TT–3' (SEQ ID NO: 18)

An HITI base sequence added to both ends of the homology arms is shown below.
5'-AGTCCGCCTAGAAGCACTTGCGG-3' (SEQ ID NO: 19)

A base sequence of foreign DNA containing the GFP gene is the same as that in Experimental Example 3.
Cas9 protein, crRNA shown in SEQ ID NO: 20

(5'-AGUCCGCCUAGAAGCACUUGGUUUUAGAGCUAUGCU-3'), tracrRNA, and the above-described donor plasmid were introduced into fertilized mouse eggs by microinjection.

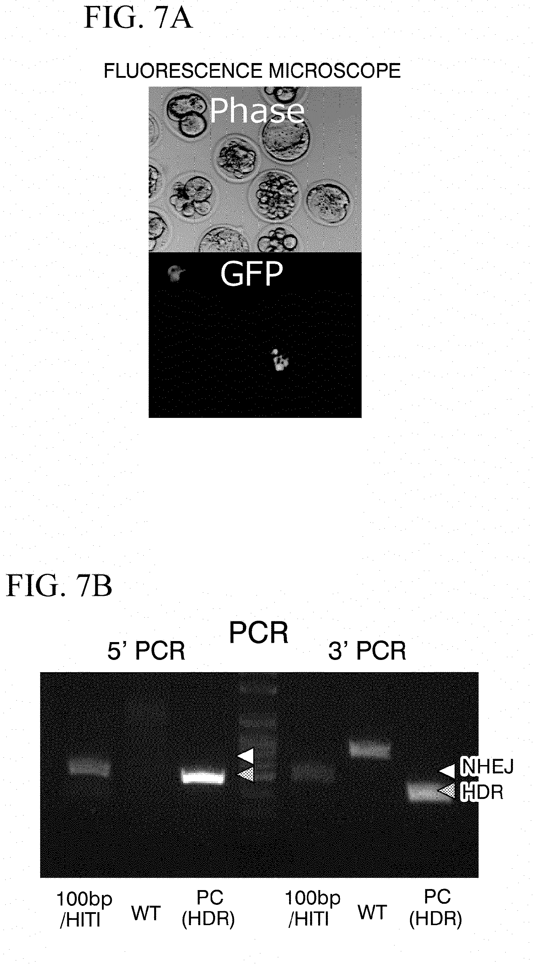

Genomic DNA was extracted from the cells 6 days after the microinjection, and DNA insertion was confirmed by PCR. As shown in Fig. 7A, the cells exhibited green fluorescence when they were observed with a fluorescence microscope, and therefore it was confirmed that the GFP gene was knocked in by homologous recombination at the 5'-end. Furthermore, as shown in Fig. 7B, at the 5'-end, a PCR band of a size corresponding to homologous recombination was recognized, and at the 3'-end, a PCR band of a size corresponding to non-homologous recombination was recognized. That is, the GFP gene was knocked in in the fertilized mouse egg by lateral homologous recombination. This results indicate that a length of the homology arm does not necessarily have to be different at both ends in the case of lateral homologous recombination.

### <Experimental Example 5>

A donor plasmid containing homology arms, each with about 60 bp, at both ends, and a donor plasmid containing homology arms, each with about 240 bp, at both ends were produced for knocking in a GFP gene at an HPRT locus of a human T-cell leukemia cell (Jurkat cell) genome.

A base sequence containing the homology arm with 60 bp at a 5'-end is shown below.

5'–GATGAACCAGGTTATGACCTTGATTTATTTTGCATACCTAATCATTAT
GCTGAGGATTTG–3' (SEQ ID NO: 21)

Abase sequence containing the homology arm with 244 bp at a 5'-end is shown below.

5’–CCGGCCTGTTGTTTTCTTACATAATTCATTATCATACCTACAAAGTTA
ACAGTTACTAATATCATCTTACACCTAAATTTCTCTGATAGACTAAGGTTATTTT
TTAACATCTTAATCCAATCAAATGTTTGTATCCTGTAATGCTCTCATTGAAACAG
CTATATTTCTTTTTCAGATTAGTGATGATGAACCAGGTTATGACCTTGATTTATTT
TGCATACCTAATCATTATGCTGAGGATTTG–3’ (SEQ ID NO: 22)

A base sequence containing the homology arm with 61 bp at a 3'-end is shown below.

5’-GAAAGGGTGTTTATTCCTCATGGACTAATTATGGACAGGTAAGTAA
GATCTTAAAATGAGG-3’ (SEQ ID NO: 23)

A base sequence containing the homology arm with 239 bp at a 3'-end is shown below.

5'–GAAAGGGTGTTTATTCCTCATGGACTAATTATGGACAGGTAAGTAA
GATCTTAAAATGAGGTTTTTACTTTTCTTGTGTTAATTTCAAACATCAGCAG
CTGTTCTGAGTACTTGCTATTTGAACATAAACTAGGCCAACTTATTAAATAACT
GATGCTTTCTAAAATCTTCTTTATTAAAAATAAAAGAGGAGGGCCTTACTAATT
ACTTAGTATCAGTTGTGGTATAGTGGGACTC–3' (SEQ ID NO: 24)

A base sequence of foreign DNA containing a GFP gene is shown below.

5'-gaattcATGGTGAGCAAGGGCGAGGAGCTGTTCACCGGGGTGGTGCC
CATCCTGGTCGAGCTGGACGGCGACGTAAACGGCCACAAGTTCAGCGTGTCC
GGCGAGGGCGAGGGCGATGCCACCTACGGCAAGCTGACCCTGAAGTTCATCT
GCACCACCGGCAAGCTGCCCGTGCCCTGGCCCACCCTCGTGACCACCCTGAC
CTACGGCGTGCAGTGCTTCAGCCGCTACCCCGACCACATGAAGCAGCACGAC
TTCTTCAAGTCCGCCATGCCCGAAGGCTACGTCCAGGAGCGCACCATCTTCTT
CAAGGACGACGGCAACTACAAGACCCGCGCCGAGGTGAAGTTCGAGGGCGA
CACCCTGGTGAACCGCATCGAGCTGAAGGGCATCGACTTCAAGGAGGACGGC
AACATCCTGGGGCACAAGCTGGAGTACAACTtCAACAGCCACAACGTCTATAT
CATGGCCGACAAGCAGAAGAACGGCATCAAGGcGAACTTCAAGATCCGCCAC
AACATCGAGGACGGCAGCGTGCAGCTCGCCGACCACTACCAGCAGAACACCC

CCATCGGCGACGGCCCCGTGCTGCTGCCCGACAACCACTACCTGAcCACCCAG
TCCGCCCTGAGCAAAGACCCCAACGAGAAGCGCGATCACATGGTCCTGgTGG
AGTTCGTGACCGCCGCCGGGATCACTCTCGGCATGGACGAGCTGTACAAGTA
Aggatcc–3' (SEQ ID NO: 25)

An HITI base sequence added to both ends of the homology arms is shown below.
5'-ACCCTTTCCAAATCCTCAGCATAATG-3' (SEQ ID NO: 26)

A plasmid (px330-HPRT) co-expressing Cas9 protein and sgRNA having a recognition sequence shown in SEQ ID NO: 27 (5'-UUAUGCUGAGGAUUUGGAAA-3'), and the above-described donor plasmid were introduced into the Jurkat cells by electroporation.

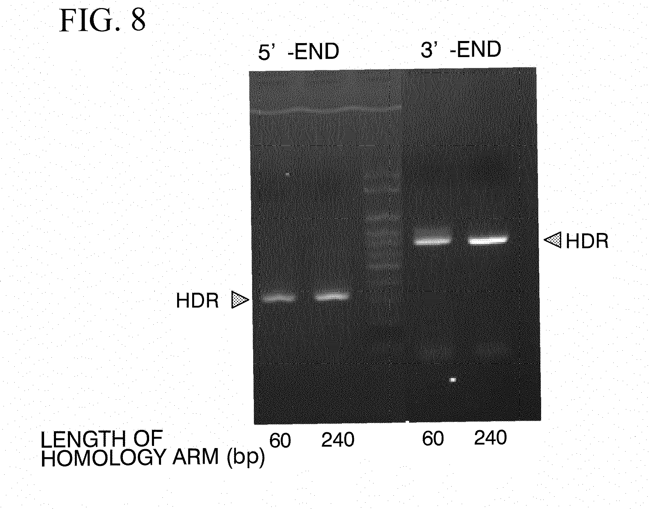

Genomic DNA was purified from the cells 3 days after the electroporation, and DNA insertion was confirmed by PCR. As shown in Fig. 8, bands of a size corresponding to homologous recombination at the 5'-end and the 3'-end were recognized. That is, knock-in by bilateral homologous recombination was recognized in the human T-cell leukemia cells. It was confirmed by the sequence as follows that the knock-in was due to error-free homologous recombination.

A TA-cloned PCR product was introduced into Escherichia coli, and each colony formed was sequenced. In a case of the arm with 60bp, it was confirmed that homologous recombination occurred in all clones of 8 clones at the 5'-end, and homologous recombination occurred in all clones of 7 clones at the 3'-end. In a case of the arm with 240bp, it was confirmed that homologous recombination occurred in all clones of 6 clones at the 5'-end, and homologous recombination occurred in all clones of 8 clones at the 3'-end.

### <Experimental Example 6>

A donor plasmid containing homology arms, each with 100 bp, at both ends was produced for knocking in a GFP gene at an LMNB1 locus of a human embryonic kidney cell line (HEK293T) genome.

A base sequence containing the homology arm with 101 bp at a 5'-end is shown below.

5’–CGCCGGTTTGTGCCTTCGGTCCCCGCTTCGCCCCCTGCCGTCCCCT
CCTTATCACGGTCCCGCTCGCGGCCTCGCCGCCCCGCTGTCTCCGCCGCCCGC
CA–3’ (SEQ ID NO: 28)

A base sequence containing the homology arm with 101 bp at a 3'-end is shown below.

5'–acCCCCGTGCCGCCGCGGATGGGCAGCCGCGCTGGCGGCCCCACCA

CGCCGCTGAGCCCCACGCGCCTGTCGCGGCTCCAGGAGAAGGAGGAGCTGC

GCGA–3' (SEQ ID NO: 29)

A base sequence of foreign DNA containing a GFP gene is shown below.

5’-gatcTGACAATGGTGAGCAAGGGCGAGGAGCTGTTCACCGGGGTGG
TGCCCATCCTGGTCGAGCTGGACGGCGACGTAAACGGCCACAAGTTCAGCGT
GTCCGGCGAGGGCGAGGGCGATGCCACCTACGGCAAGCTGACCCTGAAGTTC
ATCTGCACCACCGGCAAGCTGCCCGTGCCCTGGCCCACCCTCGTGACCACCCT

GACCTACGGCGTGCAGTGCTTCAGCCGCTACCCCGACCACATGAAGCAGCAC
GACTTCTTCAAGTCCGCCATGCCCGAAGGCTACGTCCAGGAGCGCACCATCTT
CTTCAAGGACGACGGCAACTACAAGACCCGCGCCGAGGTGAAGTTCGAGGG
CGACACCCTGGTGAACCGCATCGAGCTGAAGGGCATCGACTTCAAGGAGGAC
GGCAACATCCTGGGGCACAAGCTGGAGTACAACTtCAACAGCCACAACGTCTA
TATCATGGCCGACAAGCAGAAGAACGGCATCAAGGcGAACTTCAAGATCCGC
CACAACATCGAGGACGGCAGCGTGCAGCTCGCCGACCACTACCAGCAGAACA
CCCCCATCGGCGACGGCCCCGTGCTGCTGCCCGACAACCACTACCTGAcCACC
CAGTCCGCCCTGAGCAAAGACCCCAACGAGAAGCGCGATCACATGGTCCTGg
TGGAGTTCGTGACCGCCGCCGGGATCACTCTCGGCATGGACGAGCTGTACAA
GGCCGGCTCCGgtac-3' (SEQ ID NO: 30)

An HITI base sequence added to both ends of the homology arms is shown below.
5'-GGGGTCGCAGTCGCCATGGCGGG-3' (SEQ ID NO: 31)

An rcHITI base sequence added to both ends of the homology arms (reverse complement of HITI, that is, orientation of a guide RNA recognition sequence containing a PAM sequence in the same direction as a genome at both ends of foreign DNA) is shown below.
5'-CCCGCCATGGCGACTGCGACCCC-3' (SEQ ID NO: 32)

A plasmid (px330-LMNB1) co-expressing Cas9 protein and sgRNA having a recognition sequence shown in SEQ ID NO: 33 (5'-GGGGUCGCAGUCGCCAUGGC-3'), and the above-described donor plasmid were introduced by lipofection.

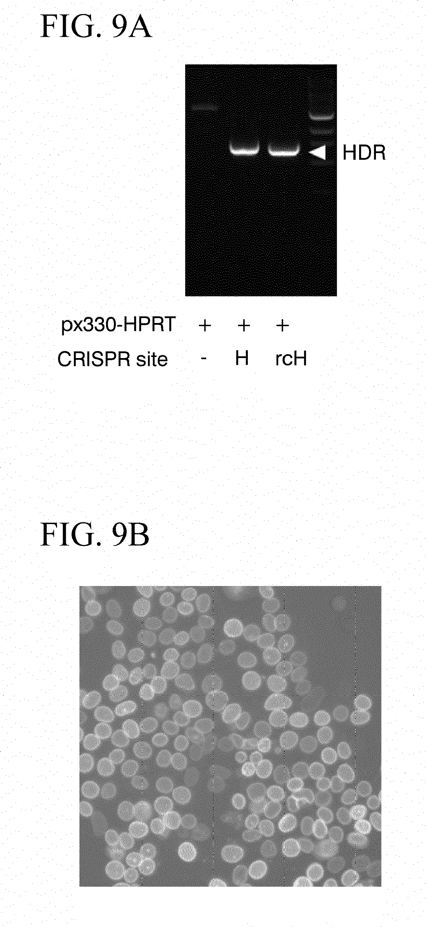

As shown in Fig. 9A, only bands corresponding to homologous recombination were recognized when the guide RNA recognition sequence of any one of HITI or rcHITI was provided.

As shown in Fig. 9B, 4 days after the lipofection, GFP-positive cells were purified by FACS to purify the GFP-positive cells. After culturing for another week, the nuclear envelope was green when it was observed with a fluorescence microscope, confirming that GFP was localized in the nuclear envelope. The above-described donor plasmid has been designed so that an LMNB1 gene and a GFP gene are fused when bilateral homologous recombination occurs and a product is localized in the nuclear envelope. Accordingly, it was confirmed that homologous recombination occurred at the 5'-end and the 3'-end from the fluorescence image.

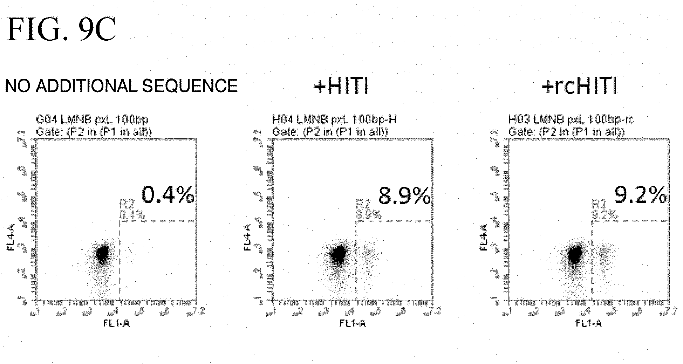

In addition, as shown in Fig. 9C, when GFP-expressing cells were quantified using flow cytometry one week after lipofection, it was confirmed that homologous recombination occurred at 8.9% to 9.2% when using the donor plasmid to which the HITI base sequence or the rcHITI base sequence was added.

### <Experimental Example 7>

A donor plasmid containing homology arms, each with about 10 bp, at both ends, a donor plasmid containing homology arms, each with about 60 bp, at both ends, and a donor plasmid containing homology arms, each with about 240 bp, at both ends were produced for knocking in a GFP gene at an HPRT locus of bone marrow stromal cells derived from a human.

A base sequence containing the homology arm with 10 bp at a 5'-end is shown below.
5'-TGAGGATTTG-3' (SEQ ID NO: 34)

A base sequence containing the homology arm with 10 bp at a 3'-end is shown below.
5'-GAAAGGGTGT-3' (SEQ ID NO: 35)

A base sequence of homology arms, each with about 60 bp to about 240 bp, at both ends, a base sequence of HITI, and a base sequence of foreign DNA are the same as those in Experimental Example 6.

A plasmid (px330-HPRT) co-expressing Cas9 protein and sgRNA having a recognition sequence shown in SEQ ID NO: 27, and the above-described donor plasmid were introduced into bone marrow stromal cells derived from a human by lipofection.

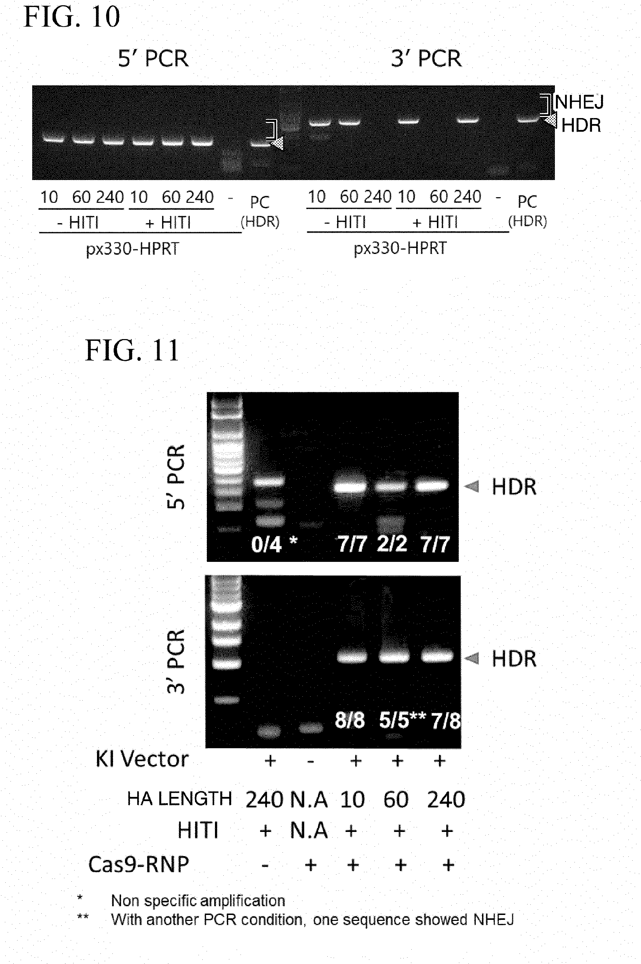

Genomic DNA was purified from the cells 3 days after the lipofection, and DNA insertion was confirmed by PCR. As shown in Fig. 10, bands of a size corresponding to homologous recombination at the 5'-end and the 3'-end were recognized. That is, knock-in in the bone marrow stromal cells derived from a human was bilateral homologous recombination.

### <Experimental Example 8>

A donor plasmid containing homology arms, each with about 10 bp, at both ends, a donor plasmid containing homology arms, each with about 60 bp, at both ends, and a donor plasmid containing homology arms, each with about 240 bp, at both ends were produced for knocking in a GFP gene at an HPRT locus of human iPS cells.

A base sequence of homology arms, each with about 10 bp to about 240 bp, at both ends, a base sequence of HITI, and a base sequence of foreign DNA are the same as those in Experimental Example 7.

In the same manner as in Experimental Example 1, Cas9 protein, crRNA shown in SEQ ID NO: 36 (5'-UUAUGCUGAGGAUUUGGAAAGUUUUAGAGCUAUGCU-3'), tracrRNA, and the above-described donor plasmid were introduced into the human iPS cells by electroporation.

Genomic DNA was purified from the cells 4 days after the electroporation, and DNA insertion was confirmed by PCR. As shown in Fig. 11, bands of a size corresponding to homologous recombination at the 5'-end and the 3'-end were recognized. In addition, homologous recombination was confirmed by sequence. That is, knock-in in the iPS cells was bilateral homologous recombination.

### <Experimental Example 9>

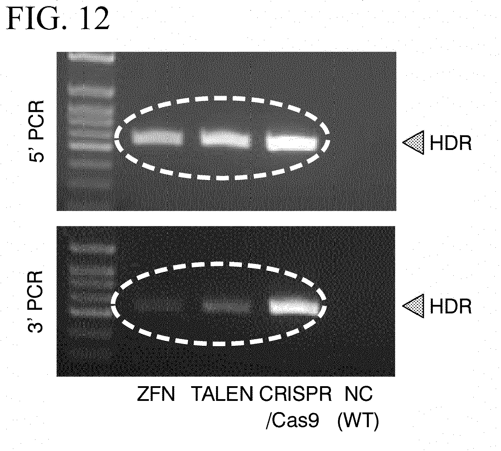

The experiment in <Experimental Example 3> was performed using ZFN and TALEN. A donor plasmid containing homology arms, each with 100 bp, at both ends was used. The results are shown in Fig. 12. Also in the case where ZFN or TALEN was used, bands of a size corresponding to homologous recombination at the 5'-end and the 3'-end were recognized. In the present invention, it has been clarified that a targeted genomic DNA-cleaving enzyme is not limited to CRISPR/Cas9, and any one of ZFN or TALEN may be used.

Table 1 shows the results of confirming the recombination method by sequence in the above experimental example as in <Experimental Example 1>. The numbers in parentheses indicate efficiency of homologous recombination.

As shown in Table 1, it was confirmed that knock-in by homologous recombination is performed regardless of animal species or target loci.

**[Table 1]**

| Species | Kind of cell | Length of homology arm (bp) | | | | | |
|---|---|---|---|---|---|---|---|
| | | 10 | 50 | 60 | 100 250 500 | | |
| Pig | Hematopoietic stem cell | 5' (6/6) | 5' (8/8) | | | | |
| | | 3' (10/10 ) | 3' (7/7) | | | | |
| Human | Jurkat | | 5' | 5' (8/8) | | 5' (6/6) | |
| | | | | 3' (7/7) | | 3' (8/8) | |
| | iPS cell | 5' (7/7) | | 5' (2/2) | | 5' (7/7) | |
| | | 3' (8/8) | | 3' (5/5) | | 3' (7/7)) | |
| Mouse | Hematopoietic stem cell | 5' (4/4) | | 5' | 5' (5/5) | | 5' (1/1) |
| | | 3' (4/4) | | | | | 3' (2/2) |
| | Fertilized egg | | | | 5' (7/7) | | |
| | | | | | 3' (0/11) | | |
| Pig | Bone marrow stromal cell | 5' (0/10) | 5' (9/9) | | | | |
| | | | 3' (1/8) | | | | |

### [Industrial Applicability]

According to the present invention, a high frequency of homologous recombination in a targeted genome can be realized without impairing non-homologous end joining, which is an inherent ability of cells, as compared with non-homologous recombination.

## Claims

1. A genome editing method which is a method for editing a genome in an isolated cell, the method comprising introducing foreign DNA into a targeted genome with homologous recombination of at least one of a 5'-end or a 3'-end of the foreign DNA when double-strand breaks of a targeted genomic DNA occur, where the foreign DNA has homology arms, each with a length of less than 500 bp, at the 5'-end and the 3'-end.

2. The genome editing method according to claim 1, wherein the foreign DNA is introduced into the targeted genome with homologous recombination of both of the 5'-end and the 3'-end of the foreign DNA.

3. The genome editing method according to claim 1 or 2, wherein the cell is a blood cell or an undifferentiated cell.

4. The genome editing method according to any one of claims 1 to 3, wherein the cell is a stem cell.

5. The genome editing method according to any one of claims 1 to 4, wherein the cell is a hematopoietic stem cell.

6. The genome editing method according to claim 1, wherein the foreign DNA is introduced into the targeted genome with homologous recombination of one of the 5'-end or the 3'-end of the foreign DNA and non-homologous recombination of the other end.

7. A genome editing method which is a method for editing a genome in an isolated cell, the method comprising introducing foreign double-stranded DNA into a targeted genome with homologous recombination of both of a 5'-end and a 3'-end of the foreign DNA, where the foreign DNA has homology arms, each with a length of less than 500 bp.

8. The genome editing method according to claim 7, wherein the cell is a blood cell or an undifferentiated cell.

9. The genome editing method according to claim 7 or 8, wherein the cell is a stem cell.

10. The genome editing method according to any one of claims 7 to 9, wherein the cell is a hematopoietic stem cell.

11. A composition comprising foreign double-stranded DNA having homology arms, each with a length of less than 500 bp, at both ends.

12. The composition according to claim 11, further comprising a targeted genomic DNA-cleaving enzyme, or DNA or mRNA encoding the enzyme.

13. The composition according to claim 11 or 12, wherein the composition is for pharmaceutical use.

14. The composition according to any one of claims 11 to 13, wherein the composition is used for treatment of severe combined immunodeficiency.

15. A method for producing a cell preparation for treating severe combined immunodeficiency, the method comprising, in a cell, introducing foreign DNA into a genome of the cell with homologous recombination of at least one of a 5'-end or a 3'-end of the foreign DNA when double-strand breaks of a targeted genomic DNA occur, where the foreign DNA has homology arms, each with a length of less than 500 bp, and has at least a part of wild-type DNA of the targeted genomic DNA.

16. The method for producing a cell preparation according to claim 15, wherein the foreign DNA is introduced into the targeted genomic DNA with homologous recombination of both of the 5'-end and the 3'-end of the foreign DNA.

17. The method for producing a cell preparation according to claim 15 or 16, wherein the cell is a blood cell or an undifferentiated cell.

18. The method for producing a cell preparation according to any one of claims 15 to 17, wherein the cell is a stem cell.

19. The method for producing a cell preparation according to any one of claims 15 to 18, wherein the cell is a hematopoietic stem cell.

20. The method for producing a cell preparation according to claim 15, wherein the foreign DNA is introduced into the targeted genomic DNA with homologous recombination of one of the 5'-end or the 3'-end of the foreign DNA and non-homologous recombination of the other end.

21. A method for producing a cell preparation for treating severe combined immunodeficiency, the method comprising, in a cell, introducing foreign DNA into a genome of the cell with homologous recombination of both of a 5'-end or a 3'-end of the foreign DNA, where the foreign DNA has homology arms, each with a length of less than 500 bp, and has at least a part of wild-type DNA of the targeted genomic DNA.

22. The method for producing a cell preparation according to claim 21, wherein the cell is a blood cell or an undifferentiated cell.

23. The method for producing a cell preparation according to claim 21 or 22, wherein the cell is a stem cell.

24. The method for producing a cell preparation according to any one of claims 21 to 23, wherein the cell is a hematopoietic stem cell.

25. A cell comprising a fragment derived from foreign DNA at a 5'-end or a 3'-end of a genome insertion site of the foreign DNA in a targeted genome of the cell.

26. A cell preparation comprising the cell according to claim 25.
